**Europäisches Patentamt**

**European Patent Office**

**Office européen des brevets**

(11) Veröffentlichungsnummer : **0 340 154 B1**

(12) ## EUROPÄISCHE PATENTSCHRIFT

(45) Veröffentlichungstag der Patentschrift :
**02.09.92 Patentblatt 92/36**

(21) Anmeldenummer : **89730107.3**

(22) Anmeldetag : **20.04.89**

(51) Int. Cl.$^5$ : **A45D 19/02, A45D 34/04**

(54) **Applikator zur Behandlung der Haut.**

(30) Priorität : **29.04.88 DE 8805927 U**

(43) Veröffentlichungstag der Anmeldung :
**02.11.89 Patentblatt 89/44**

(45) Bekanntmachung des Hinweises auf die
Patenterteilung :
**02.09.92 Patentblatt 92/36**

(84) Benannte Vertragsstaaten :
**AT BE CH DE ES FR GB GR IT LI LU NL SE**

(56) Entgegenhaltungen :
**EP-A- 0 150 651
CH-A- 304 890**

(56) Entgegenhaltungen :
**DE-A- 2 619 990
DE-A- 3 122 516
FR-A- 2 581 569
GB-A- 1 559 542
US-A- 2 906 300
US-A- 3 196 886**

(73) Patentinhaber : **SCHERING
AKTIENGESELLSCHAFT Berlin und
Bergkamen
Müllerstrasse 170/178 Postfach 65 03 11
W-1000 Berlin 65 (DE)**

(72) Erfinder : **Butterbrodt, Gerhard
Helmstedter Strasse 26
W-1000 Berlin 31 (DE)**

EP 0 340 154 B1

## Beschreibung

Die Erfindung betrifft einen Applikator nach dem Oberbegriff des Patentanspruchs 1.

Aus der DE 31 22 516 A1 ist ein Gerät bekannt, bei dem das Verschlußstück einen Druckkolben aufnimmt, mit dem das Mittel zur Behandlung der Kopfhaut aus dem Gerät herausgedrückt wird. Der Zylinderinnenraum muß bei offenem Gerät durch Einkippen oder Eindrücken des Behandlungsmittels gefüllt werden. Eine genaue Dosierung des Mittels ist nicht möglich.

Die deutsche Gebrauchsmusterschrift 18 03 642 beschreibt ein Gerät zum Auftragen von Stoffen auf den Haarboden. Dieses Gerät ist auf eine Vorratsflasche aufschraubbar. Eine exakte Dosierung des Behandlungsmittels ist nicht möglich.

Der Erfindung liegt die Aufgabe zugrunde, einen Applikator der vorstehend beschriebenen Art zu schaffen, der als Handgerät verwendet und mit dem eine genau dosierte Menge eines Behandlungsmittels abgegeben werden kann. Diese Forderung ist insbesondere bei der Applikation von Medikamenten wesentlich, die zu bestimmten Zeiten in genau vorbestimmten Dosen erfolgen soll.

Erfindungsgemäß wird diese Aufgabe durch die im Anspruch 1 enthaltene technische Lehre gelöst.

Mit besonderem Vorteil begrenzt das Verschlußstück im Zylinder einen Zylinderinnenraum mit einem festen, vorbestimmten Volumen und weist eine zentrale Füllöffnung auf, an der eine Rücklaufsperre ausgebildet ist. Es ist möglich, unter Verwendung einer Vorratsflasche oder einer Vorratstube den Zylinderinnenraum mit einer genau dosierten Menge des Behandlungsmittels zu füllen. Die Rücklaufsperre verhindert nach der Füllung ein Auslaufen des Behandlungsmittels.

Die erfindungsgemäße Ausbildung des Applikators erlaubt dessen Anbringung an einem Vorratsbehälter. Weiterhin besteht mit Vorteil die Möglichkeit, nach der Füllung des Applikators diesen von Vorratsbehälter zu trennen und als Handgerät zu verwenden.

Mit Vorteil liegt das Volumen des vom Verschlußstück abgeschlossenen Zylinderinnerraums im Bereich von 0,1 bis 5 ml, insbesondere 0,25 bis 2,5 ml.

Bei einer vorteilhaften Ausführungsform ist die Rücklaufsperre als Rücklaufsperrstutzen ausgebildet. Dieser Rücklaufperrstutzen erstreckt sich von der zentralen Füllöffnung in den Zylinderinnenraum hinein und verhindert nach einer Füllung und nach einem Umdrehen des Applikators ein Auslaufen.

Bei einer anderen vorteilhaften Ausführungsform des Applikators ist ein Ventilsitz an der Füllöffnung ausgebildet, der von einem Käfig umgeben ist, in dem ein freifliegender Ventilkörper angeordnet ist. Mit besonderem Vorteil ist das Ventil als Kegelventil ausgebildet.

Da für die Hautbehandlung vorgesehene Kosmetika oder Pharmazeutika häufig in Fläschchen, insbesondere Kunststofffläschchen geliefert werden, die einen Pump- oder Spendermechanismus aufweisen, ist mit besonderem Vorteil der Applikator derart ausbildbar, daß dieser zusammen mit derartigen Spende- oder Vorratsgefäßen verwendet werden kann. Zu diesem Zweck ist am Verschlußstück mindestens ein formflüssiger Paßsitz für ein Vorratsgefäß oder eine Vorratsflasche ausgebildet.

Mit Vorteil ist das Verschlußstück als in die offene Stirnseite des Zylinders einfügbar ausgebildet. Das Verschlußstück kann beispielsweise durch Aufprellen oder Ultraschallschweißung mit dem Zylinder fest verbunden sein. Bei einer anderen Ausführungsform weist das Verschlußstück ein Außengewinde und der Zylinder ein Innengewinde auf.

Zur besseren Handhabung ist am Umfang des Zylinders eine Rändelung vorgesehen.

Zum Schutz des Applikators ist eine auf den Zylinder aufsetzbare Schutzkappe vorgesehen.

Der Applikator weist einen, an der Stirnseite offenen Zylinder auf, der mittels eines Verschlußstückes verschließbar ist. Innerhalb des Zylinders wird auf diese Weise eine Vorratskammer gebildet. An der Stirnwand des Zylinders sind axial sich erstreckende Voll- und Hohlzapfen ausgebildet. Die Hohlzapfen münden in den Zylinderinnenraum und weisen an der Spitze eine öffnung auf. Bei Verwendung dieses Applikators können in dem Zylinder enthaltene Medikamente oder Kosmetika durch die Hohlzapfen hindurch auf die zu behandelnde Haut abgegeben werden und durch eine entsprechende Massagebewegung mittels der am Zylinder angeordneten Vollzapfen in die Haut einmassiert werden. Durch Bemessung der Querschnitte der Hohlzapfen können für bestimmte Medikamte oder Kosmetika Dosierungen eingestellt werden.

Mit Vorteil sind die Voll- und Hohlzapfen kegelförmig ausgebildet.

Zur Erhöhung der Stabilität sind in den Radialebenen die Durchmesser der Vollzapfen kleiner gestaltet, als die der Hohlzapfen. Zur Erzielung einer guten Massagewirkung sind die Spitzen der Vollzapfen abgerundet.

In vorteilhafter Weise sind die Spitzen der Hohlzapfen abgerundet und die Öffnung ist als Kreuzspalt ausgebildet. Ein besonders günstiger Behandlungseffekt wird dadurch erzielt, daß jeweils Voll- und Hohlzapfen an der Stirnwand abwechselnd in konzentrischen Kreisen angeordnet sind. Dabei ist der Kreis größten Durchmessers (der Randkreis) mit vollzapfen besetzt und im Mittelpunkt der Stirnwand ist ein Hohlzapfen angeord-net.

2

Zur Anpassung an Wölbungen der Hautoberfläche, insbesonder im Bereich der Kopfhaut, liegen die Spitzen der Voll- und Hohlzapfen auf einer Hüllkugelfläche.

Ausführungsbeispiele der Erfindung sollen in der folgenden Beschreibung unter Bezugnahme auf die Figuren der Zeichnung erläutert werden.

Es zeigen:

Fig. 1 eine Schnittansicht des Zylinders des Applikators,

Fig. 2 eine Draufsicht auf den in Fig. 1 dargestellten Zylinder,

Fig. 3A u. 3B eine Schnittansicht und eine Draufsicht einer Ausführungsform des Verschlußstückes für den Zylinder,

Fig. 4A - 4C Darstellungen eines Verschlußstückes mit Rückschlagventil,

Fig. 5 eine vergrößerte Darstellung konstruktiver Einzelheiten des Zylinders und

Fig. 6 eine Schnittansicht einer Schutzkappe.

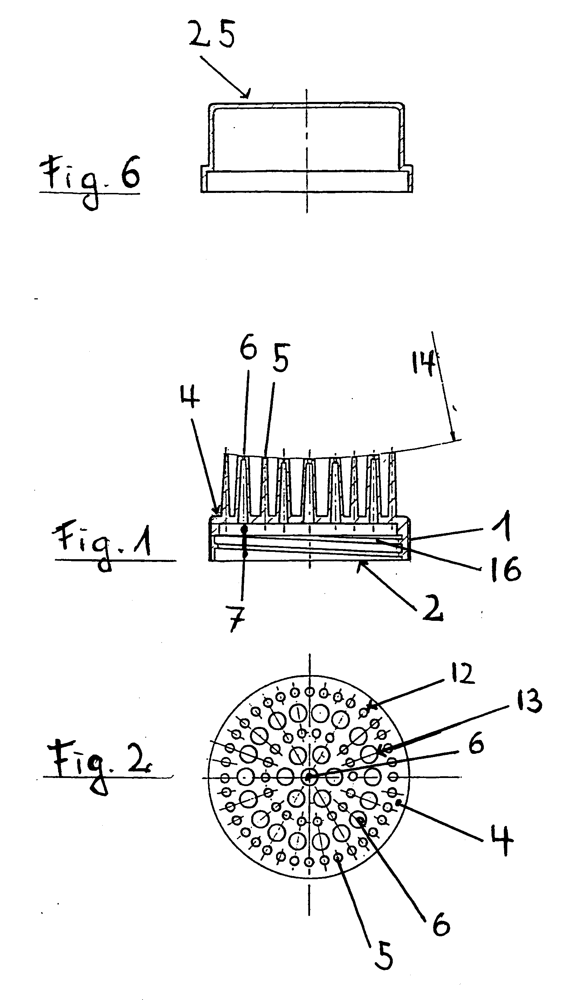

Wie Fig. 1 zeigt, ist ein an der Stirnseite 2 offener Zylinder 1 vorgesehen. Von der Stirnwand 4 des Zylinders erstrecken sich axial Vollzapfen 5 und Hohlzapfen 6.

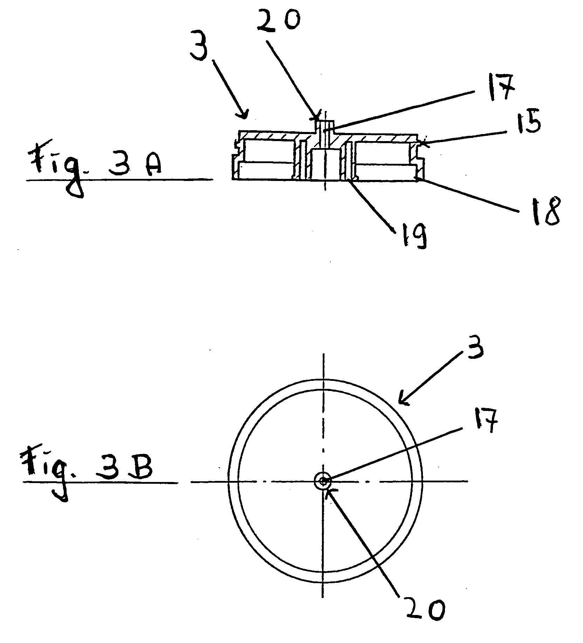

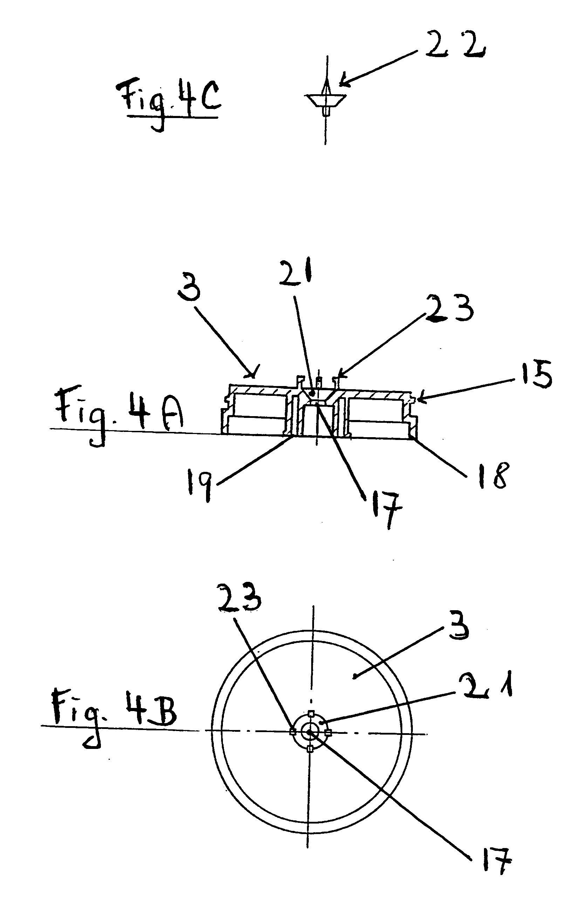

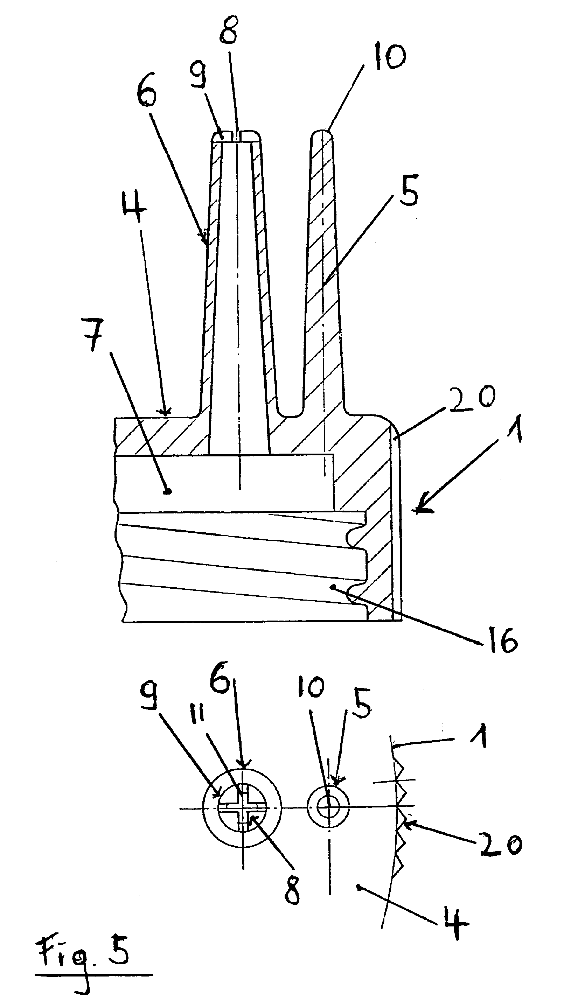

Die offene Stirnseite 2 des Zylinders ist mittels eines Verschlußstückes 3 verschließbar, von dem Ausführungsbeispiele in den Fig. 3 A und 3 B und 4 A - 4 C dargestellt sind. Dieses Verschlußstück 3 kann mit dem Zylinder 1 fest und unlösbar verbunden sein. Bei den dargestellten Ausführungsbeispielen weist der Zylinder 1 ein Innengewinde 16 und die Ausführungsbeispiele des Verschlußstückes 3 weisen ein Außengewinde 15 auf, so daß diese beiden Teile miteinander verschraubt werden können. Zwischen diesen Teilen verbleibt dann der in den Fig. 1 und 5 dargestellte Hohlraum 7.

Wie insbesondere der Fig. 5 zu entnehmen ist, münden die Hohlzapfen 6 in den Vorrats- oder Hohlraum 7 und weisen an ihren Spitzen 9 eine Öffnung 8 auf. Im Hohl- oder Vorratsraum 7 befindliche Kosmetika oder Pharmazeutika können bei Verwendung des Applikators durch die Hohlzapfen 6 auf die zu behandelnden Hautabschnitte austreten und werden durch eine Massage-Bewegung mittels der Vollzapfen 5 in die Haut einmassiert.

Wie Fig. 2 zeigt, sind die Vollzapfen 5 und die Hohlzapfen 6 abwechselnd in konzentrischen Kreisen angeordnet. Der äußere Kreis 12 ist mit Vollzapfen 5 und der darauf folgende Kreis 13 mit Hohlzapfen 6 besetzt. In der Mitte befindet sich ein Hohlzapfen 6.

Wie Fig. 1 zeigt, weisen die Voll- und Hohlzapfen unterschiedliche Längen auf. Die Ausbildung ist derart, daß die Spitzen aller Voll- und Hohlzapfen auf einer Kugelhüllfläche liegen, die schematisch in Fig. 1 bei 14 angedeutet ist.

Das in den Fig. 3 A und 3 B dargestellte Verschlußstück 3 weist in der Mitte eine Füllöffnung 17 auf. Weiterhin sind am Verschlußstück 3 Paßsitzfassungen 18 und 19 vorgesehen. Wie die Fig. 3 A und 3 B zeigen, ist die Füllöffnung 17 von einer Rücklaufsperrbuchse 20 umgeben. Diese Sperrbuchse 20 verhindert, daß bei der Verwendung des Applikators in den Zylinderinnenraum 7 abgegebene Flüssigkeit wieder durch die Füllöffnung 17 auslaufen kann.

Bei dem in den Fig. 4 A - 4 C dargestellten Verschlußstück 3 ist zur Verhinderung einer Rückströmung ein Rückschlagventil vorgesehen. Wie die Fig. 4 A und 4 B zeigen, ist am Mündungsende der Füllöffnung 17 ein Kegelventilsitz 21 ausgebildet. Um diesen Kegelventilsitz 21 herum sind mehrere Kegelventil-Käfighaken 23 angeordnet. Diese Käfighaken 23 schließen zusammen mit dem Kegelventilsitz 21 einen Käfig ein. In diesem Käfig ist der Fig. 4 C dargestellte Ventikegel 22 freifliegend angeordnet.

Wie Fig. 5 zeigt, ist die Öffnung 8 an der Spitze 9 der Hohlzapfen 6 als Kreuzspalt 11 ausgebildet. Die Spitzen 10 der Vollzapfen 5 sind, wie ebenfalls der Fig. 5 zu entnehmen ist, abgerundet. Der Fig. 5 ist ferner zu entnehmen, daß die Voll- und die Hohlzapfen eine Kegelform aufweisen. In den gleichen Radialebenen sind die Durchmesser der Hohlzapfen 6 größer als die der Vollzapfen 5.

Fig. 5 zeigt die Ausbildung einer Rändelung 20 am Umfang des Zylinders 1.

Bei Verwendung von Kunststofflaschen, die ein Medikament oder ein Pharmazeutikum enthalten und die einen Pump- oder Spendermechanismus aufweisen, kann das Verschlußstück 3 im Paßsitz das Abgabeende dieser Kunststofflasche aufnehmen. Zur Anwendung ist es erforderlich, den Zylinder 1 auf das Verschlußstück 3 aufzuschrauben.

Fig. 6 zeigt die Schnittansicht einer Verschlußkappe 25, die auf den in Fig. 1 dargestellten Zylinder 1 aufgesteckt werden kann, um insbesondere die Spitzen der Voll- und Hohlzapfen 5 und 6 zu schützen.

## Patentansprüche

1.    Applikator zur Behandlung der Haut, insbesondere der Kopfhaut mit pharmazeutischen und kosmetischen Wirkstoffen mit

einem an der Stirnseite offenen Zylinder,

einem Verschlußstück für dessen offene Stirnseite,

von der Stirnwand des Zylinders axial sich erstreckenden Voll- und Hohlzapfen, von denen

die Hohlzapfen in den zylinderraum münden und

eine Öffnung an der Spitze aufweisen,

dadurch gekennzeichnet, daß

das Verschlußstück (3) im Zylinder (1) einen Zylinderinnenraum (7) mit festem, vorbestimmtem Volumen begrenzt, und

eine zentrale Füllöffnung (17) aufweist, an der

eine Rücklaufsperre (20, 21, 22, 23) ausgebildet ist.

2.  Applikator nach Anspruch 1,
dadurch gekennzeichnet, daß

das Volumen des vom Verschlußstück (3) abgeschlossenen Zylinderinnenraumes (7) im Bereich von 0,1 bis 5 ml, insbesondere 0,25 bis 2,5 ml liegt.

3.  Applikator nach Anspruch 1 oder 2,
dadurch gekennzeichnet, daß

die Rücklaufsperre als Rücklaufsperrstutzen (20) ausgebildet ist.

4.  Applikator nach Anspruch 1 oder 2,
gekennzeichnet durch

einen Ventilsitz (21) an der Füllöffnung (17), der

von einem Käfig (23) umgeben ist, in dem

ein freifliegender Ventilkörper (22) angeordnet ist.

5.  Applikator nach Anspruch 4,
dadurch gekennzeichnet,

daß das Ventil (21, 22) ein Kegelventil ist.

6.  Applikator nach einem der Ansprüche 1 - 5,
dadurch gekennzeichnet, daß

am Verschlußstück (3) mindestens ein formschlüssiger Paßsitz (18, 19) für ein Vorratsgefäß oder eine -flasche ausgebildet ist.

7.  Applikator nach einem der Ansprüche 1 - 6,
dadurch gekennzeichnet, daß

das Verschlußstück (3) als in die offene Stirnseite (2) des Zylinders (1) einfügbar ausgebildet ist.

8.  Applikator nach einem der Ansprüche 1 - 7,
dadurch gekennzeichnet, daß

das Verschlußstück (3) mit dem Zylinder (1) fest verbunden ist.

9.  Applikator nach einem der Ansprüche 1 - 8,
dadurch gekennzeichnet, daß

das Verschlußstück (3) ein Außengewinde (15) und

der Zylinder (1) ein Innengewinde (16) aufweisen.

10. Applikator nach einem der Ansprüche 1 - 9,
dadurch gekennzeichnet, daß

am Umfang des Zylinders (1) eine Rändelung (20 A) ausgebildet ist.

11. Applikator nach einem der Ansprüche 1 -10,
gekennzeichnet durch

ein Schutzkappe (25) für den Zylinder (1).

## Claims

1. Applicator for the treatment of the skin, especially the scalp, with pharmaceutical and cosmetic active substances, comprising
a cylinder that is open at the end face,
a closure piece for the open end face of the cylinder,
solid and hollow pins extending in the axial direction from the end wall of the cylinder, of which
the hollow pins open into the cylindrical chamber and have an opening at their tips,
characterised in that
the closure piece (3) in the cylinder (1) defines a cylindrical inner chamber (7) of a fixed, predetermined volume and
has a central filling opening (17) at which
there is constructed a non-return means (20, 21, 22, 23).

2. Applicator according to claim 1, characterised in that the volume of the cylindrical inner chamber (7) closed by the closure piece (3) is in the region of from 0.1 to 5 ml, especially from 0.25 to 2.5 ml.

3. Applicator according to claim 1 or 2, characterised in that the non-return means is constructed in the form of a non-return connection piece (20).

4. Applicator according to claim 1 or 2, characterised by a valve seat (21) located at the filling opening (17) and surrounded by a cage (23) in which there is arranged a free-floating valve body (22).

5. Applicator according to claim 4, characterised in that the valve (21, 22) is a cone valve.

6. Applicator according to any one of claims 1 to 5, characterised in that there is constructed on the closure piece (3) at least one positively locking snug-fit seat (18, 19) for a supply container or a supply bottle.

7. Applicator according to any one of claims 1 to 6, characterised in that the closure piece (3) is constructed to be insertable into the open end face (2) of the cylinder (1).

8. Applicator according to any one of claims 1 to 7, characterised in that the closure piece (3) is rigidly connected to the cylinder (1).

9. Applicator according to any one of claims 1 to 8, characterised in that the closure piece (3) has an external thread (15) and the cylinder (1) has an internal thread (16).

10. Applicator according to any one of claims 1 to 9, characterised in that a knurled surface (20A) is constructed around the circumference of the cylinder (1).

11. Applicator according to any one of claims 1 to 10, characterised by a protective cap (25) for the cylinder (1).

## Revendications

1. Applicateur pour le traitement de la peau, notamment de la peau du crâne au moyen de substances pharmaceutiques et cosmétiques, comportant
un cylindre ouvert au niveau de sa face frontale,
un élément de fermeture pour cette face frontale ouverte,
des tiges pleines et creuses qui s'étendent axialement à partir de la paroi frontale du cylindre et parmi lesquelles les tiges creuses débouchent dans l'espace du cylindre, et comportent une ouverture au niveau de leur pointe,
caractérisé par le fait que
l'élément de fermeture (3) limite, dans le cylindre (1), un espace intérieur cylindrique (16) possédant un volume fixe prédéterminé, et
possède une ouverture de remplissage centrale (17), dans laquelle
est installé un dispositif de blocage antiretour (20,21,22, 23).

2. Applicateur selon la revendication 1, caractérisé en ce que
le volume de l'espace intérieur cylindrique (7), fermé par l'élément de fermeture (3), se situe dans une gamme comprise entre 0,5 et 5 ml et notamment entre 0,25 et 2,5 ml.

3. Applicateur selon la revendication 1 ou 2, caractérisé en ce que le dispositif de blocage antiretour est réalisé sous la forme d'un embout de blocage antiretour (20).

4. Applicateur selon la revendication 1 ou 2, caractérisé par
un siège de soupape (21) installé sur l'ouverture de remplissage (17) et qui est entouré par une cage (23) dans laquelle est disposé un corps de soupape flottant (22).

5. Applicateur suivant la revendication 4, caractérisé en ce que la soupape (21,22) est une soupape à pointeau.

6. Applicateur suivant l'une des revendications 1-5, caractérisé en ce que sur l'élément de fermeture (3) est prévu au moins un système d'ajustement serré (18,19), par formes complémentaires, pour une bouteille ou un récipient formant réservoir.

7. Applicateur selon l'une des revendications 1-6, caractérisé en ce que l'élément de fermeture (3) est agencé de manière à pouvoir être inséré dans la face frontale ouverte (2) du cylindre (1).

8. Applicateur selon l'une des revendications 1-7, caractérisé en ce que l'élément de fermeture (3) est raccordé rigidement au cylindre (1).

9. Applicateur selon l'une des revendications 1-8, caractérisé en ce que l'élément de fermeture (3) possède un filetage extérieur (15) et que le cylindre (1) possède un taraudage (16).

10. Applicateur selon l'une des revendications 1-9, caractérisé en ce qu'un moletage (20 A) est formé sur le pourtour du cylindre (1).

11. Applicateur selon l'une des revendications 1-10, caractérisé par un capot de protection (25) pour le cylindre (1).

25

Fig. 6

6  5

4                    14

Fig. 1                              1
                                    16
7                            2

Fig. 2

12

13

6

6

4

5

7

Fig. 3 A

Fig. 3 B

Fig.4C

22

Fig. 4 A

3  21  23  15  19  17  18

Fig. 4B

23  3  21  17

Fig. 5